# EUROPEAN PATENT SPECIFICATION

(11) **EP 2 934 407 B1**
(45) Date of publication and mention of the grant of the patent: **09.08.2017**
(21) Application number: 12881983.6
(22) Date of filing: 20.12.2012
(51) Int. Cl.: A61F 13/505, A61F 13/475, A61F 13/551

(54) **AN ABSORBENT ARTICLE COMPRISING AT LEAST TWO STACKED INDIVIDUAL ABSORBENT PADS**
SAUGFÄHIGER ARTIKEL MIT MINDESTENS ZWEI ÜBEREINANDER ANGEORDNETEN EINZELNEN SAUGFÄHIGEN EINLAGEN
ARTICLE ABSORBANT COMPRENANT AU MOINS DEUX COUSSINETS ABSORBANTS INDIVIDUELS EMPILÉS

(43) Date of publication of application: 28.10.2015
(73) Proprietor: SCA Hygiene Products AB, 405 03 Göteborg (SE)
(72) Inventor: DAHL, Dennis, 405 03 Göteborg (SE); BURVALL, Angelica, 405 03 Göteborg (SE)
(74) Representative: Zacco Sweden AB
(86) International application number: PCT/SE2012/051464
(87) International publication number: WO 2014/098680

(56) References cited:
- EP-A1- 0 549 988
- EP-A1- 0 767 644
- EP-A2- 0 861 641
- WO-A1-03/072003
- JP-A- H0 938 136
- JP-A- H0 938 136
- JP-A- H1 028 701
- JP-A- 2002 336 296
- JP-A- 2003 175 073
- JP-A- 2010 201 093
- US-A- 5 820 616
- US-A- 5 820 616
- US-A1- 2003 199 844
- US-A1- 2005 027 278

## Description

### TECHNICAL FIELD

This invention relates to an absorbent article, such as an incontinence pad, a sanitary towel, a panty liner or the like, comprising at least two stacked individual absorbent pads, a first of the absorbent pads is configured for being releasably attached to an underwear of a user, and a bottom surface of a second of the absorbent pads is configured for being releasably attached to a top surface of the first absorbent pad. The invention also relates to a method of manufacturing an absorbent article having stacked individual absorbent pads.

### BACKGROUND OF THE INVENTION

Absorbent articles are commonly used for the absorption of body fluids, including but not limited to, infant diapers and training pants, adult incontinence products, feminine hygiene products, gender specific absorbent products, and pet training absorbent articles. The main requirement of such an absorbent article is absorbing body fluids discharged from the wearer, but the absorbent article should also provide the wearer with a feeling of comfort and freshness.

Absorbent articles used as feminine products are commonly discarded once the absorbent material is saturated with fluids such as menstrual blood. In certain situations, however, there might be a hassle to bring extra absorbent articles and it might further be inconvenient to change the absorbent article. In these situations it is beneficial to use an absorbent article comprising multiple stacked individual absorbent pads that may be separately removed as part of the article gets soiled.

A multilayer absorbent article is disclosed in EP0767644 A1. The document discloses a multilayer absorbent feminine hygiene product with a plurality of, preferably three, absorbent pads. The first layer of the absorbent article is releasably attached to the wearer's undergarment, and the additional layers are releasably attached to the first layer and to each other. During use, the uppermost pad layer may be peeled of when soiled, thereby exposing an unused underlying pad layer. Handling and disposal of the absorbent article during removal of one or more additional layers is however unsatisfactory with respect to freshness, hygiene and simplicity.

JP H09 38136 A discloses an absorbent article comprising two stacked individual absorbent pads.

US5820616 A provides an absorbent article which includes a first absorbent article and a second absorbent article releasably secured to one another. The second absorbent article is folded along its longitudinal edges to provide side flaps which are releasably secured to the garment facing surface of the first absorbent article.

US 2003/199844 disclose an absorbent article comprising a replaceable absorbent core component disposed in capillary liquid communication with a non-removable absorbent core compartment.

There is thus a need for an improved absorbent article removing the above mentioned disadvantage.

### SUMMARY OF THE INVENTION

An object of the present invention is to provide an inventive absorbent article comprising at least two stacked individual absorbent pads where the previously mentioned problem is at least partly avoided. It is also an object of the present invention to provide a manufacturing method for a corresponding absorbent article. These objects are achieved by the features of the independent claims.

The invention concerns an absorbent article, such as an incontinence pad, a sanitary towel, a panty liner or the like, comprising at least two stacked individual absorbent pads. A first of the absorbent pads is configured for being releasably attached to an underwear of a user, and a bottom surface of a second of the absorbent pads is configured for being releasably attached to a top surface of said first absorbent pad.

The invention is characterized in that the second absorbent pad comprises at least one side portion which has been folded upwardly and inwardly along a longitudinal fold line, such as to form at least one easily graspable gripping flap for simplifying manual handling of the second absorbent pad during removal thereof from the first absorbent pad.

Advantages of the inventive solution comprise easiness of use. The gripping flap facilitates easy gripping of the second absorbent pad, the one being removed to expose the first absorbent pad for further use, and hence easy removal of the second absorbent pad from the first absorbent pad. Without the gripping flap, the user may have difficulties in identifying and gripping the second absorbent pad for removal thereof. Furthermore, the gripping flap also enables a reduced degree of soiling of the hands during removal of the second absorbent pad, thereby enabling increased level of freshness and hygiene. Ease of handling and removal of a second absorbent pad is beneficial in situations where the user of the absorbent article is in a location where changing of absorbent article is awkward or in a situation where the user do not have extra absorbent articles available. The second absorbent pad may be discarded after removal from the underlying first absorbent pad, and the gripping tab may also simplify disposal of the second absorbent pad in that the second absorbent pad may be folded and possibly at least partly secured in a folded state by means of the gripping flap. The side portion of the first absorbent pad is before use preferably free from upwardly folded side portions to enable a simplified separation of the side portions of the first and second absorbent pads.

Further advantages are achieved by implementing one or several of the features of the dependent claims.

The first absorbent pad may comprise at least a liquid permeable top sheet, a liquid impermeable back sheet and an absorbent core enclosed therebetween. The first absorbent pad may also be provided with one or more additional layers, such as additional absorbent layers, acquisition and/or distribution layers, forming elements or stiffening elements.

The second absorbent pad may comprise at least a liquid permeable top sheet such that body fluids may be absorbed through the pad. The liquid permeable top sheet further provides the user with a sense of dryness. The second absorbent pad may additionally comprise a liquid permeable back sheet, or a liquid impermeable back sheet, or a semi-liquid permeable back sheet. A liquid permeable back sheet or a semi-liquid permeable back sheet provides further sense of dryness to the user and reduces the likelihood for leakage, because body fluids may be distributed from the second absorbent pad to the first absorbent pad. This aspect is particularly advantageous if the second absorbent pad has a relatively low absorption capacity. A liquid impermeable back sheet of the second absorbent pad may on the other hand facilitate that the first absorbent pad is unsoiled and dry when the second absorbent pad is removed from the first absorbent pad by use of the gripping flaps. Thereby, the first absorbent pad still has full absorption capacity when the second absorbent pad is removed.

In order to provide a better absorption capacity of the second absorbent pad, the second absorbent pad may additionally comprise an absorbent core enclosed between the top sheet and back sheet. The absorbent core is preferably thin such that the second absorbent pad does not significantly contribute to a thick and uncomfortable absorbent article to wear but still provides excellent absorption properties.

Preferably, the gripping flap is formed by a top sheet of the second absorbent pad, or by a back sheet of the second absorbent pad, or by at least both a top and back sheet of the second absorbent pad. These materials are relatively thin and thus easily foldable and do not contribute much to the thickness of the folded gripping flaps. Thereby the stacked absorbent article remains relatively thin and comfortable to wear.

In order to ensure that the purpose of the gripping flaps is easily understood by the user and that the second absorbent pad is removed by grasping and pulling the gripping flaps, the function and intended handling of the gripping flaps may be clarified and made obvious by providing the top surface of the first and/or second absorbent pad with perforation, printing, colouring or patterns, and/or by providing the gripping flap with a special folding or special shape. By these means the user is taught and instructed that the gripping flaps should be grasped and pulled to remove the second absorbent pad. The visual impression of the gripping flaps should however preferably not impair the impression of function of the absorbent article nor the function itself.

The gripping flap may be strengthened by means of a tape, film, string and/or coating applied to at least part of an inner and/or outer surface of the gripping flap. Thereby, deforming e.g. by creasing, of the gripping flaps during transport, handling and wearing is reduced which facilitates the grabbing of the gripping flaps as well as reduces risk of discomfort for the wearer of the absorbent article due to deformation of the flaps of the article. The strengthening of the gripping flaps also facilitates the correct folding of the flaps.

The gripping flap is preferably detached from the first absorbent pad, i.e. the gripping flap itself does not adhere or is otherwise directly fastened to the first absorbent pad, other than by the indirect fastening via the rest of the second absorbent pad and its attachment to the top sheet of the first absorbent pad. The gripping flap of the second absorbent pad is thereby easily separable from the first absorbent pad, and each of the gripping flap and the first absorbent pad may thus be easily grasped by a separate hand of a user during removal of the second absorbent pad.

The gripping flap may be at least partly liquid impermeable and configured to have dual functionality in terms of gripping flap for simplified handling of the second absorbent pad and side barrier for reducing side leakage of body fluids, such as menstrual blood or urine. Side leakage herein represents leakage towards the longitudinal sides of the absorbent article. The liquid impermeability of the gripping flap may be realised by applying a liquid impermeable tape, film, string and/or coating to at least part of an inner and/or outer surface of the gripping flap, and/or by forming the gripping flap at least partly by a liquid impermeable top sheet or back sheet of the second absorbent pad.

At least the top sheets of each absorbent pad of the absorbent article may further be provided with grooves, channels, apertures or indentations for further improving the side leakage barrier, but also other underlying layers of each absorbent pad may exhibit such grooves, etc. Substantially longitudinally arranged grooves, channels or indentations enable body fluids to spread in the longitudinal direction rather than in lateral direction. By adding grooves etc. to at least the top sheet body fluids are retained in the central portion of the absorbent article and do not leak in a lateral direction. Risk of soiling the undergarment is thereby reduced. The second absorbent pad and the first absorbent pad may both be provided with grooves or the like. Grooves, channels, apertures or indentations may also give the visual impression of a side leakage barrier. If both pads are provided with grooves or the like, it is preferred that the grooves of the second absorbent pad and the grooves of the first absorbent pad provide the absorbent article with a uniform visual appearance. Moreover, the grooves etc. of the first and/or second absorbent pad are preferably combined with the gripping flap to generate a uniform visual appearance.

The first absorbent pad may extend beyond a side edge of the second absorbent pad in a transverse direction in a region of the at least one gripping flap, such that both the first and second absorbent pads are easily graspable during removal of the second absorbent pad from the first absorbent pad. Thereby, the first and second absorbent pads are easily separable, grasped and pulled apart when removing the second absorbent pad.

The first absorbent pad may also extend beyond a side edge of the second absorbent pad along the entire periphery of the absorbent article. In this aspect of the invention, the second absorbent pad is smaller than the first absorbent pad in both the longitudinal and transversal direction of the absorbent article. The absorbent article is thus a stacked article with individual pads in the centre but remains thin at the periphery where only the first absorbent pad extends. The thin periphery increases the comfort of wearing the absorbent article as it is relatively thin at the periphery where the article commonly meets the tendons of the user's thighs. The thickness of individual first and second absorbent pads may of course vary over their respective surface, and they may for example have thicker central region, or having a uniform thickness.

The absorbent article may be folded along a first and a second lateral fold line that divide the absorbent article in three, preferably substantially equally large, parts for providing a smaller and more portable article, wherein the second absorbent pad has a maximal longitudinal extension that is smaller than half the maximal longitudinal extension of the first absorbent pad and arranged to fit in a central region of said absorbent article without being folded along said lateral fold lines. The absorbent article may be folded along lateral fold lines for reducing the size thereof and enabling a more transportable article. By ensuring that the second absorbent pad is not folded along a lateral fold line the folded absorbent article remains relatively thin. Would also the second absorbent pad be folded along one or two lateral fold lines the folded absorbent article would obviously be thicker. The longitudinal position of the first and a second lateral fold line may vary according to the specific configuration of the absorbent article. However, when considering only the size of the folded article, then the first and second lateral fold lines are preferably positioned such that the absorbent article is divided in three substantially equally large parts. As an alternative, only the core layer of the second absorbent pad may be formed to fit within the lateral fold lines and the relatively thin top and/or back sheet may be allowed to extend beyond the lateral fold line as their thickness does not significantly results in a thicker folded absorbent article. Furthermore, when the second absorbent pad lacks a separate absorbent core and the top and/or back sheet or other thin absorbent sheet functions as absorbent, then the longitudinal extension of the second absorbent pad is less relevant due to the relatively thin shape thereof.

A side edge of the first absorbent pad may overlap the side edge of the second absorbent pad along the entire periphery of the absorbent pads. This configuration corresponds to an absorbent article where the first and second absorbent pads have same longitudinal and lateral extension over the entire pad, i.e. an absorbent article where no absorbent pad extends beyond the other absorbent pad at any position.

The first and second absorbent pads may be mutually releasably connected by means of fastening means, in particular pressure-sensitive adhesive, ultrasonic processing, hooks and loops, thermal bonding, mechanical bonding such as punching or embossing, or the like. The type of fastening means and position thereof is preferably selected to ensure that the top surface of the first absorbent pad is not damaged or impaired during separation of the second absorbent pad from the first absorbent pad. This is particularly important when the fastening means is positioned in an expected wetting area of the first absorbent pad, i.e. a relatively centrally arranged area of the first absorbent pad. When the fastening means only are located in a region adjacent the periphery of the first absorbent pad, then a certain degree of damages to top surface of the first absorbent article may be acceptable.

The first absorbent pad may have two laterally spaced apart wing sections that extend beyond the laterally opposite sides of the absorbent core of the first absorbent pad, wherein each of the wing sections is arranged to be folded around a side edge of the crotch portion of the undergarment for securing the first absorbent pad to the undergarment and/or preventing soiling thereof. The wings may preferably be fastened to the undergarment by fastening means such as glue or hook-and-loop fastener or similar suitable fastening means.

The invention also concern a method for manufacturing an absorbent article, such as an incontinence pad, a sanitary towel, a panty liner or the like, having at least a first and a second stacked individual absorbent pads, wherein the second absorbent pad is provided with at least one side portion which has been folded upwardly and inwardly along a longitudinal fold line, such as to form an easily graspable gripping flap for simplifying manual handling of the second absorbent pad during removal thereof from the first absorbent pad.

In a first embodiment the method may comprise the steps of laminating a pre-cut second absorbent pad on top of a first absorbent web, releasably attaching a bottom surface of the pre-cut second absorbent pad to a top surface of the first absorbent web; and cutting out the first absorbent pad from the first absorbent web, such as to form a stacked absorbent article. The method may further comprise the step of folding the at least one side portion of the second absorbent pad before laminating the pre-cut second absorbent pad on top of the first absorbent web. This method enables a relatively large difference in size of the first and second absorbent pad, as well as large freedom in relative positioning of the first and second absorbent pads.

In another embodiment the method may comprise the steps of laminating a second absorbent web on top of a first absorbent web, releasably attaching a bottom surface of the second absorbent web to a top surface of the first absorbent web and cutting out the first and second absorbent pads from the laminated absorbent webs, such as to form a stacked absorbent article. One benefit of this embodiment is that an erroneous positioning of a second absorbent pad onto the first absorbent web is eliminated as the cutting of the first and second absorbent pads are performed simultaneously after releasably attaching the second web onto the first web. Production costs will also be reduced due to the elimination of the separate cut-out of the second absorbent pad. The method may further comprise the step of folding at least one lateral side of the second absorbent web before laminating the folded second absorbent web on top of the first absorbent web. Folding the lateral side of the second absorbent web before lamination simplifies the folding step because a potential interference with the first absorbent pad is avoided.

The terms transverse direction and lateral direction are synonyms. Upward herein means in a direction from the first absorbent pad towards the second absorbent pad, i.e. towards a user in a usage position. Inward herein means a substantially lateral direction towards a longitudinal centre line of the article. The side edge of an absorbent pad is the edge of the absorbent pad in a lateral direction. The side portion of an absorbent pad herein refers to at least a portion of the lateral side of the absorbent pad, i.e. a surface area of the absorbent pad positioned adjacent and extending to the side edge of the absorbent pad. An absorbent core means a member having a good absorption capacity. An absorbent core may have a limited extension in the absorbent pad, i.e. only extending in a central area of the absorbent pad and not at the edges thereof. An absorbent core may however equally be formed by an absorbent layer extending completely or partly over the absorbent pad. The term "member" does not limit the number of parts forming the member to a single part. A member may for example be formed of two separate parts.

### BRIEF DESCRIPTION OF DRAWINGS

In the detailed description of the invention given below reference is made to the following figure, in which:
- Figure 1: shows a cross section view of an absorbent article according to a first embodiment of the invention;
- Figure 2: shows a top view of an absorbent article of fig. 1;
- Fig 3a-3c: show different cross section views of a folded gripping flap of the second absorbent pad of an absorbent article according to the invention;
- Figure 4a: shows a top view of second embodiment of the absorbent article according to the invention;
- Figure 4b: shows a cross-sectional view according to cut A-A in fig. 4a.
- Figure 5a: shows a top view of an absorbent article according to third embodiment of the invention;
- Figure 5b: shows a cross-sectional view according to cut A-A in fig. 5a.

### DESCRIPTION OF EXAMPLE EMBODIMENTS OF THE INVENTION

Various aspects of the invention will hereinafter be described in conjunction with the appended drawings to illustrate and not to limit the invention, wherein like designations denote like elements, and variations of the inventive aspects are not restricted to the specifically shown embodiments, but are applicable on other variations of the invention.

The present invention pertains to an absorbent article. An absorbent article is defined as an article or garment used for the absorption of body fluids, including but not limited to, infant diapers and training pants, adult incontinence products, feminine hygiene products, gender specific absorbent products, and pet training absorbent articles. While the figures do not illustrate every type of absorbent article, it should be understood that the present invention pertains to all types of absorbent articles.

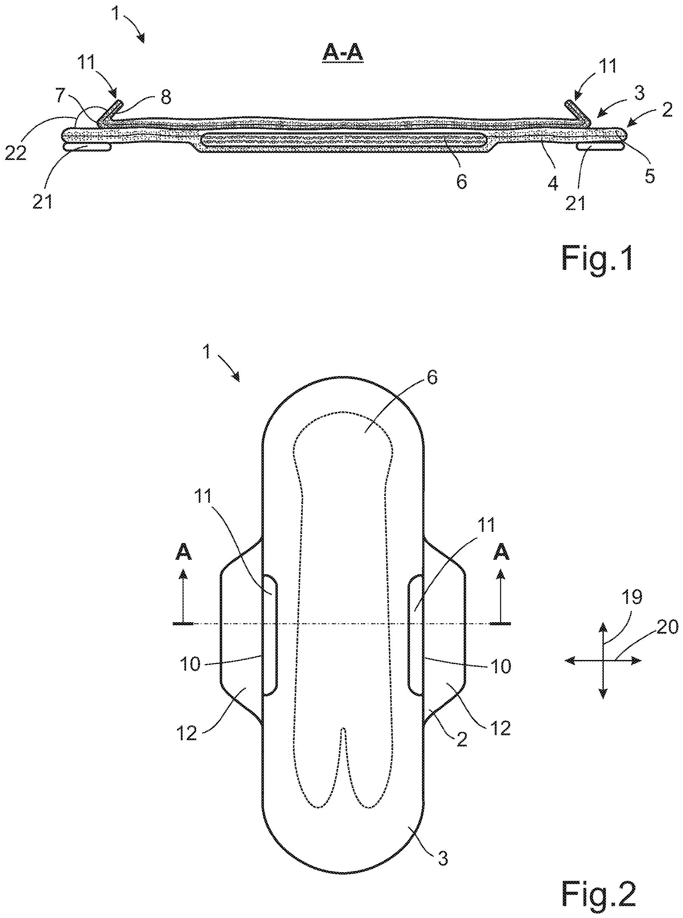

FIG. 1 and 2 show a first embodiment of the absorbent article according to the invention. The absorbent article 1 includes first absorbent pad 2 and a second absorbent pad 3. The first and second absorbent pads 2, 3 are arranged stacked, i.e. overlappingly, and the first absorbent pad 2 is a lower pad and the second absorbent pad 3 is an upper pad. The absorbent article 1 is elongated in a longitudinal direction 19, which is perpendicular to a traverse direction 20. The first absorbent pad 2 comprises a back sheet 4, a top sheet 5, and an absorbent core 6 sandwiched between the back sheet 4 and top sheet 5. The second absorbent pad 3 comprises a back sheet 7 and a top sheet 8. The second absorbent pad 3 is free from a separate absorbent core in this embodiment, and at least the top sheet 8 functions as an absorbing member to a certain degree. The bottom surface of the back sheet 7 of the second absorbent pad 3 is releasably attached to the top surface of the top sheet 5 of the first absorbent pad 2.

The back sheet 7 of the second absorbent pad 3 and the top sheet 5 of the first absorbent pad 2 are mutually releasably connected by means of fastening means (non-showed). Fastening means may include pressure-sensitive adhesive, ultrasonic processing, hooks and loops, thermal bonding, mechanical bonding such as punching or embossing, or the like. Suitable fastening means include fastening means that does not disrupt or impair the properties, pattern and/or structure of the top layer 5 of the first absorbent pad 2 when the second absorbent pad 3 is removed. Thereby, the functionality of the first absorbent pad 2 is intact and may be used after removal of the second absorbent pad 3. Further, the fastening means ensure that the second absorbent pad 3 does not move in relation to the first absorbent pad 2 during wear of the absorbent article 1. If hooks and loops or pressure-sensitive adhesive is used for mutual attachment of the first and second absorbent pads 2, 3, then this fastening means may also be used for closing the second absorbent pad 3 during disposal thereof, such as simplified and improved handling of the absorbent article is realised.

The second absorbent pad 3 may have a different thickness and/or absorption capacity compared with the first absorbent pad 2. For example, the second absorbent pad 3 may be thinner or thicker than the first absorbent pad 2, measured on an average thickness of each absorbent pad 2, 3. The first absorbent pad 2 normally has a higher absorption capacity than the second absorbent pad 3, but also this aspect may be opposite. The materials in the back sheet 7 and top sheet 8 are selected depending on the needs and requirements of the second absorbent pad 3. A relatively thin second absorbent pad 3 formed without a separate absorbent core and having a relatively low absorption capacity may comprise a relatively thick, at least 60 Grammes per Square Metre (gsm), liquid-permeable top sheet 8 made of a non-woven material and a back sheet 7 made of a non-woven material or a plastic material. A second absorbent pad 3 having an intermediate thickness and absorption capacity may comprise a liquid-permeable non-woven top sheet 8, non-woven back sheet 7 and a relatively thin absorbent core (non-showed embodiment). An even thicker second absorbent pad 3 having a relatively high absorption capacity may comprise a relatively thick liquid-permeable top sheet 8, a relatively thick absorbent core and a non-woven back sheet 7.

Additionally, the first and/or second absorbent pad 2, 3 may include a separate acquisition and distribution layer (non-showed) located between the top sheet 5 and the absorbent core 6. The acquisition and distribution layer is specially designed to increase the fluid acquisition rate of the absorbent pad, as well as distributing the acquired fluid over a large surface of the underlying absorbent core 6. The acquisition and distribution layer may be in the form of a polymeric film or a non-woven material, such as for example fluff pulp, airlaid nonwoven material highloft material or the like. The acquisition and distribution layer may be formed from polypropylene or polyethylene. Particular non-woven materials used for the acquisition distribution layer include spunbond-meltblown-spunbond (SMS) non-woven.

The top sheet 5, 8 of the first and/or second absorbent pads 2, 3 is liquid-permeable and includes one or more layers of one or more of the following materials: a fibrous material, for example a soft nonwoven material, plastic film, mesh, open-celled foam, material laminate etc. The top sheet 5, 8 may include a perforated plastic film, for example, a thermoplastic plastic material such as polyethylene or polypropylene, or a mesh-like layer of synthetic or textile material. Synthetic mono-, bi-, or multi-component fibres, made of polymers such as polyethylene, polypropylene, polyester, nylon or the like, are preferably used as a nonwoven material. Mixtures of different types of fibres can also be used for the aforementioned nonwoven material. The top sheet 5, 8 may also comprise fluff pulp, airlaid nonwoven material, highloft nonwoven material, superabsorbent polymers (SAP), or other absorbent increasing materials. Grooves, channels, recess, indentations or the like may be imprinted in the upper surface of the top sheet 5, 8 for improved liquid acquisition performance and reduced side leakage. If no separate acquisition and distribution layer is provided then the top sheet itself preferably has properties for functioning as acquisition and distribution layer.

The absorbent core 6 of the first absorbent pad 2, and also of the second absorbent pad 3 if an absorbent core is provided therein, is appropriately manufactured from a suitable fibre material in the form of natural or synthetic fibres forming a web having absorbent properties, or a mixture of natural fibres and synthetic fibres. The absorbent core 6 is preferably made of an airlaid nonwoven material. The absorbent core 6 can also contain a predetermined proportion of SAP. SAP materials are in the form of particles, fibres, flakes or the like, and have the capacity to absorb and to chemically bind liquid equivalent to several times their own weight while forming an aqueous gel. This provides a very high liquid-absorbent capacity in the finished product.

The back sheet 7 of the second absorbent pad 3 is preferably a non-woven as it provides a soft and flexible feeling during wear, and consequently an increased comfort. The non-woven back sheet 7 may also be calcium carbonate treated in order to further increase the softness of the non-woven material. A non-woven back sheet 7 also improves the mutual fastening of the first and second absorbent pads 2, 3 because a non-woven back sheet 7 results in a relatively high friction when positioned on a non-woven top sheet 5 of the first absorbent pad 2. The back sheet 7 may alternatively be formed by glue applied to the back surface of material layer facing the first absorbent pad 2. The back sheet 7 may be permeable, semi-permeable or non-permeable depending on the need and requirement of the second absorbent pad 3. If the second absorbent pad 3 comprises a relatively large absorption capacity the back sheet 7 of the second absorbent pad 3 may be non-permeable in order to reveal an unsoiled first absorbent pad 2 after removal of the second absorbent pad 3. A semi-permeable or permeable back sheet 7 may however for example suitable be used in combination with a second absorbent pad 3 having a relatively low absorption capacity in order to avoid leakage, because acquired fluid will start to pass from the second absorbent pad 3 to the first absorbent pad 2 when the fill level of the second absorbent 3 pad is high.

The back sheet 4 of the first absorbent pad 2 is preferably liquid-impermeable (or at least possesses high resistance to penetration by liquid) and is thus so arranged as to prevent any leakage of excreted fluid from the product. The back sheet 4, on the other hand, may also be vapour-permeable. The back sheet 4 may be manufactured from a liquid-impermeable material which includes a thin and liquid-proof plastic film. For example, plastic films of polyethylene, polypropylene or polyester can be used for this purpose. Alternatively, a laminate of nonwoven and plastic film or other suitable layers of material can be used as a liquid-proof back sheet 4. In a previously disclosed manner, the clothing side of the back sheet 4 can be provided with beads of adhesive or some other previously disclosed attachment means, which can then be utilized for the application of the product to an item of clothing i.e. undergarment.

The first absorbent pad 2 of the absorbent article 1 as shown in FIG 2 is provided with wings 12, that is to say folding flaps which are arranged along the sides of the article and can be utilized in conjunction with fastening of the absorbent article 1 onto an undergarment. The wings 12 are suitably provided with adhesive coating 21.

With reference to FIGS. 1 and 2, the second absorbent pad 3 comprises two side portions which have been folded upwardly and inwardly along a longitudinal fold line 10, and thereby forming easily graspable gripping flaps 11. The side portions may form part of wings of the second absorbent pad 3. The gripping flaps 11 facilitate simple manual handling of the second absorbent pad 3 during removal from the first absorbent pad 2. The gripping flaps 11 are folded and exhibit preferably in a natural state an angle 22 of more than 0 degrees, preferably more than 90 degrees, such that the gripping flaps are easily grasped.

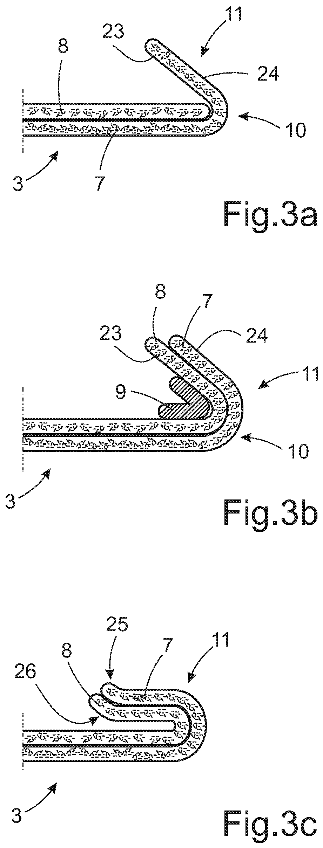

In FIG 1 the gripping flaps 11 are formed by at least both a top sheet 8 and back sheet 7 of the second absorbent pad 3. The flaps 11 may also be formed by merely the back sheet 7 of the second absorbent pad 3, as shown in FIG. 3a or merely the top sheet 8 (not shown), or by any combination of layers of the second absorbent pad 3.

In order to strengthen and improve the form stability of the folded gripping flaps 11 they may be provided with means of tape, film and/or coating applied to at least part of an inner surface 23 and/or outer surface 24 of the gripping flaps 11. They may also or alternatively be provided with absorbent tape or strings including SAP for improved side leakage protection. A gripping flap 11 strengthened with coating/glue 9 applied to the inner surface 23 of a gripping flap 11 in the location of the fold line 10 is shown in FIG 3b. Alternatively, the material of the back sheet 7 itself may be selected to provide a form stable folded gripping flap 11. Strengthening means are provided in order to facilitate easy grasping of the gripping flaps 11 even after the absorbent article 1 have been folded in a package or worn against the body during use. Strengthening means may also improve the form stability of the folded article, such that the folded gripping flaps 11 do not easily unfold, thereby potentially eliminating an advantage of the invention. The strengthening means also provide improved stability to the gripping flaps 11 when the gripping flaps 11 are arranged to additionally function as side barriers against side leakage. By applying strengthening means the gripping flaps 11 may be made thinner. The strengthening means may further indicate the position of the gripping flaps 11, and thus indicate to the user where to grasp and pull the second absorbent pad 3 during removal.

The folded gripping flaps 11 further function as side barriers in order to hinder body fluids, such as menstrual blood, to soil the side of the underlying first absorbent pad 2 or the undergarment. The folded gripping flaps 11 are preferably liquid impermeable when having dual functionality as side barriers and gripping flaps. The liquid impermeability is realised by applying a liquid impermeable tape, film, string and/or coating to at least part of an inner and/or outer surface of the gripping flap 11, or by forming the gripping flap 11 by at least a liquid impermeable or semi-impermeable back sheet 7 of the second absorbent pad 3, as illustrated in FIG. 3a. The longitudinal length of the gripping flaps 11 has influence on the side barrier leakage blocking performance. Relatively long side barriers increase the barrier performance. When the gripping flaps are intended to operate as leakage barriers, a longitudinal length of at least 50 millimetres is preferred, and a longitudinal length of at least 75 millimetres is more preferred, and a longitudinal length of at least 100 millimetres is still more preferred.

In order to further facilitate grasping of the gripping flap 11, as well as possibly improving user comfort, the outmost extending edge 25 of the gripping flap 11 may be bent along an additional longitudinal fold line 26 in the same or opposite folding direction as the fold line 10. This embodiment is shown in FIG. 3c. Grasping the folded gripping flap 11 in order to tear apart the second absorbent pad 3 and the first absorbent pad 2 may thereby be facilitated and it may also be easier for a user to intrinsically understand where to grasp the gripping flap 11. A folded side edge of the gripping flap 11 may also improve user comfort because the side edge of the gripping flap 11 may exhibit a more rounded shape.

In the article of FIG. 1 and 2, the folded gripping flaps 11 are folded inwards, i.e. towards a longitudinal centre line of the article, such that the first absorbent pad 2 extends in traverse direction 20 beyond the fold line 10 of the folded gripping flaps 11 at the periphery region of the gripping flaps 11. In FIG. 2 this extending portion of the first absorbent pad 2 constitutes the wings 12 which are intended to be folded around the crotch portion of the undergarment thereby securing the absorbent article in place. The first absorbent pad 2 extends beyond the folded gripping flaps 11 of the second absorbent pad 3 and facilitates thereby gripping of the first absorbent pad 2 when pulling to two absorbent pads 2, 3 apart.

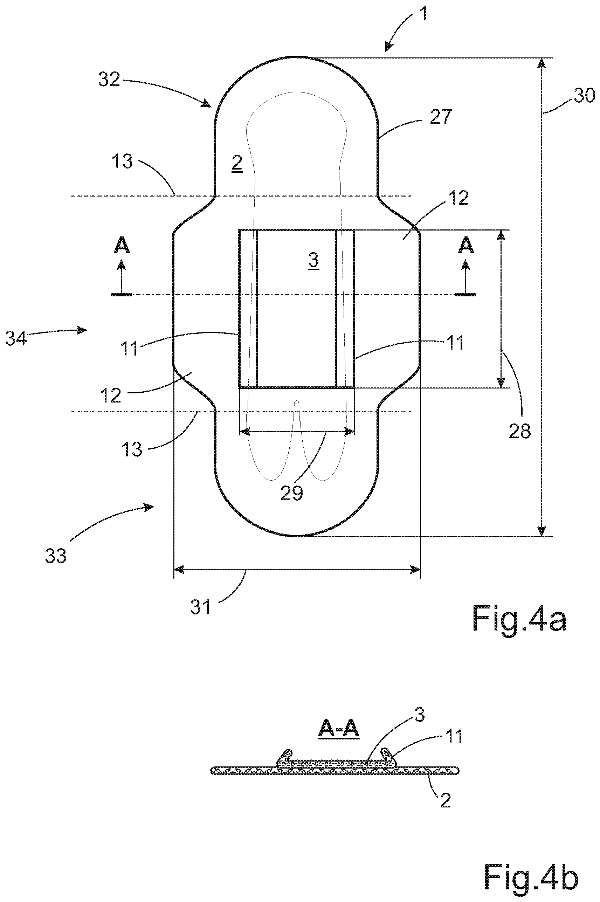

In a second embodiment of the invention as shown schematically in FIG 4a and 4b, the second absorbent pad 3 is smaller than the first absorbent pad 2, hence the first absorbent pad 2 extends beyond a side edge of the second absorbent pad 3 along the entire periphery 27 of the absorbent article 1 seen from a top view. The first absorbent pad 2 is provided with wings 12 in FIG 4a. One benefit of a smaller second absorbent pad 3 is that the absorbent article 1 is thinner in some areas of the absorbent article and thus easier to package and to transport. The perceived feeling of the absorbent article 1 is that it is thinner and less bulky when the stacked layers, e.g. the second absorbent pad 3 in this embodiment, extends less in traverse and longitudinal direction 20, 19 than the first absorbent pad 2. This may thus result in a more comfortable absorbent article. A smaller second absorbent pad 3 may also result in improved shaping properties of the absorbent article, as well as allowing simplified disposal of the second absorbent pad after removal. The longitudinal length 28 and the transverse width 29 of the second absorbent pad 3 are selected according to the specific requirements and specification of the absorbent article 1. The longitudinal length 28 of the second absorbent pad 3 may for example be less than 75% of the total longitudinal length 30 of the first absorbent pad 2, preferably less than 60%, and more preferably less than 40%. Similarly, the transverse width 29 of the second absorbent pad 3 may for example be less than 75% of the total transverse width 31 of the first absorbent pad 2, preferably less than 60%, and more preferably less than 40%.

In one beneficial embodiment the second absorbent article 3 has a maximum longitudinal extension 28 that is smaller than half the maximal longitudinal extension 30 of the first absorbent pad 2. Preferably, the second absorbent article 3 is arranged to fit in a laterally unfolded central longitudinal region 34 of the absorbent article 1, as shown in FIG 4a. This is especially beneficial during folding of the absorbent article 1 along a first and second lateral fold lines 13 that divide the absorbent article 1 in three approximately equally large parts, i.e. having approximately equal longitudinal extension, in order to package the absorbent article 1. Upon packaging of the flat article, the longitudinal end portions 32, 33 of the article 1 are folded along the lateral fold lines 13 to face the central region 34. Since the second absorbent pad 3 is located completely within the fold lines 13, i.e. within the central region 34, the second absorbent pad is not laterally folded. Thereby, the packaged absorbent article 1 is both thin and small and non-bulky. However, the absorbent article 1 may also be packaged in other ways suitable for an absorbent article 1 e.g. by folding the absorbent article laterally once or three times, or longitudinal folding, or rolling.

The absorbent article 1 shown in FIG. 4a and 4b may be manufactured by the steps of cutting second absorbent web along a cutting shape to form the second absorbent pad 3, folding at least one lateral side portion of the cut-out second absorbent pad 3 wherein folding may be performed before or after said cutting, laminating a pre-cut and pre-folded second absorbent pad 3 on top of a first absorbent web, cutting the first absorbent web along a cutting shape to form the finished absorbent article. Cutting of the first and second absorbent webs are thus performed in separate steps.

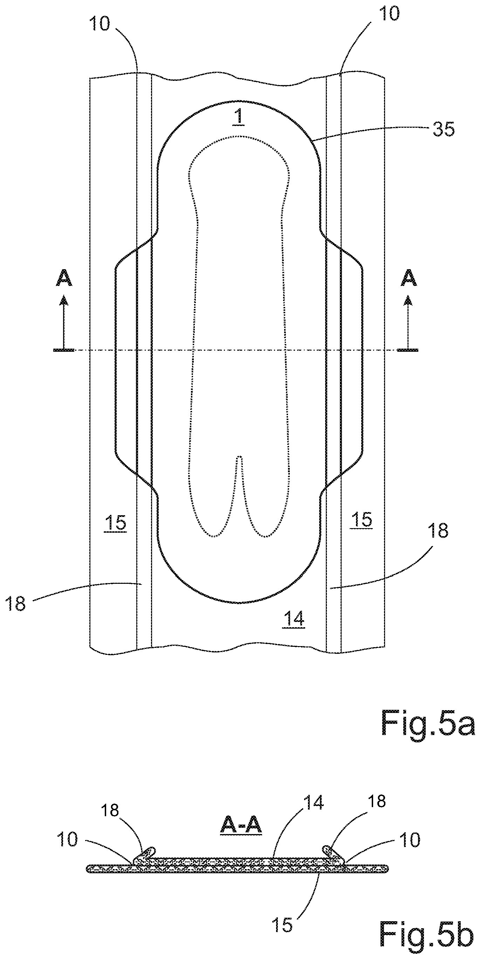

FIG. 5a and 5b show a third embodiment of the present invention in an intermediate process state of manufacturing, i.e. in a non-finished manufacturing state. A first absorbent web 15 is disclosed having a second absorbent web 14 laminated on top of the first absorbent web 15, wherein the bottom surface of the second absorbent web 14 has been releasably attached to a top surface of the first absorbent web 15. The absorbent article 1 in FIG. 5a and 5b is shown at a stage of the manufacturing method prior to cutting out the first and second absorbent pads 2, 3 from the laminated absorbent webs 14, 15. The cutting shape 35 that will be used for forming the finished absorbent article is illustrated in FIG. 5a.

The two lateral side portions 18 of the second absorbent web 14 have been folded upwardly and inwardly along two parallel longitudinal fold lines 10 such as to form the two gripping flaps 11 of the cut-out absorbent article 1.The step of folding the lateral side portions 18 of the second absorbent web 14 is preferably realised before laminating the folded second absorbent web 14 on top of the first absorbent web 15 in order to simplify the folding process step. Furthermore, since the cutting shape 35 extends beyond the fold lines 10 of the second absorbent web 14, the resulting first absorbent pad 2 will extend laterally beyond the gripping flaps 11, thereby automatically creating the desired difference in lateral extension of the first and second absorbent pad 2, 3 for simplified gripping and handling during removal of the second absorbent pad 3.

As will be realised, the invention is capable of modification in various obvious respects, all without departing from the scope of the appended claims. Various aspects of each embodiment may be combined with the various aspects of the other embodiments. The absorbent article has been disclosed having wings 12 but the invention is equally applicable for non-winged absorbent articles. The second absorbent pad 3 may also be provided with a single gripping tab 11, the gripping tab 11 may extend beyond the first absorbent pad, and the gripping tab 11 may have the same lateral extension as the first absorbent pad, although none of these embodiments have been explicitly shown in the figures. The finished absorbent article is preferably provided with colourings, prints, and/or patterned embossing for creating an attractive visual appearance of the absorbent article, as well as an educational aspect in form of indicating to the user where the gripping flaps are located and their purpose.

Reference signs mentioned in the claims should not be seen as limiting the extent of the matter protected by the claims, and their sole function is to make claims easier to understand. The drawings and the description thereto are to be regarded as illustrative in nature, and not restrictive.

## Claims

1. An absorbent article (1), such as an incontinence pad, a sanitary towel, a panty liner or the like, comprising at least two stacked individual absorbent pads (2, 3), a first of said absorbent pads (2) is configured for being releasably attached to an underwear of a user, and a bottom surface of a second of said absorbent pads (3) is configured for being releasably attached to a top surface of said first absorbent pad (2) by means of fastening means, in particular pressure-sensitive adhesive, ultrasonic processing, hooks and loops, thermal bonding, mechanical bonding such as punching or embossing, or the like, **characterised in that** the second absorbent pad (3) comprises at least one side portion which has been folded upwardly and inwardly along a substantially longitudinal fold line (10), such as to form an easily graspable gripping flap (11) for simplifying manual handling of the second absorbent pad (3) during removal thereof from the first absorbent pad (2), wherein the gripping flap (11) is detached from the first absorbent pad (2).

2. The absorbent article (1) according to claim 1, **characterised in that** the first absorbent pad (2) comprises at least a liquid permeable top sheet (5), a liquid impermeable back sheet (4) and an absorbent core (6) enclosed therebetween.

3. The absorbent article (1) according to any of the preceding claims, **characterised in that** the second absorbent pad (3) comprises at least a liquid permeable top sheet (8).

4. The absorbent article (1) according to claim 3, **characterised in that** the second absorbent pad (3) additionally comprises a liquid permeable back sheet (7), or a liquid impermeable back sheet (7), or a semi-liquid permeable back sheet (7).

5. The absorbent article (1) according to claim 4, **characterised in that** the second absorbent pad (3) additionally comprises an absorbent core enclosed between the top sheet (8) and back sheet (7).

6. The absorbent article (1) according to any of the preceding claims, **characterised in that** the gripping flap (11) is formed by a top sheet (8) of the second absorbent pad (3), or by a back sheet (7) of the second absorbent pad (3), or by at least both the top sheet (8) and the back sheet (7) of the second absorbent pad (3).

7. The absorbent article (1) according to any of the preceding claims, **characterised in that** the gripping flap (11) is strengthened by means (9) of a tape, film, string and/or coating applied to at least part of an inner and/or outer surface of the gripping flap (11).

8. The absorbent article (1) according to any of the preceding claims, **characterised in that** the gripping flap (11) is liquid impermeable and having dual functionality in terms of gripping flap (11) and side barrier for reducing side leakage of body fluids, such as menstrual blood or urine.

9. The absorbent article (1) according to claim 8, **characterised in that** the liquid impermeability of the gripping flap (11) is realised by applying a liquid impermeable tape, film, string and/or coating to at least part of an inner and/or outer surface of the gripping flap (11), or by forming the gripping flap (11) by at least a liquid impermeable back sheet (7) of the second absorbent pad (3).

10. The absorbent article (1) according to any of the preceding claims, **characterised in that** the first absorbent pad (2) extends beyond a side edge of said second absorbent pad (3) in a transverse direction in a region of said at least one gripping flap (11), such that also the first absorbent pad (2) is easily graspable during removal of the second absorbent pad (3) from the first absorbent pad (2).

11. The absorbent article (1) according to any of the preceding claims, **characterised in that** the first absorbent pad (2) extends beyond a side edge of said second absorbent pad (3) along the entire periphery of said absorbent article (1).

12. The absorbent article (1) according to any of the preceding claims, **characterised in that** the absorbent article (1) is folded along a first and a second lateral fold line (13) that divide the absorbent article (1) in three, preferably substantially equally large, parts for providing a smaller and more portable article, wherein the second absorbent pad (3) has a maximal longitudinal extension that is smaller than half the maximal longitudinal extension of the first absorbent pad (2) and arranged to fit in a central region of said absorbent article (1) without being folded along said lateral fold lines (13).

13. The absorbent article (1) according to any of the preceding claims 1 - 9, **characterised in that** a side edge of the first absorbent pad (2) overlaps the side edge of the second absorbent pad (3) along the entire periphery of the absorbent pads (2, 3).

14. The absorbent article (1) according to any of the preceding claims, **characterised in that** the first absorbent pad (2) has two spaced apart wing (12) sections that extend beyond the laterally opposite sides of the absorbent core (6) of the first absorbent pad (2), wherein each of said wing (12) sections is arranged to be folded around a side edge of the crotch portion of the undergarment for securing the first absorbent pad (2) to the undergarment and preventing soiling thereof.

15. Method for manufacturing an absorbent article (1), such as an incontinence pad, a sanitary towel, a panty liner or the like, having at least a first and a second stacked individual absorbent pads (2, 3), wherein the second absorbent pad (3) is provided with at least one side portion which has been folded upwardly and inwardly along a substantially longitudinal fold line (10), such as to form an easily graspable gripping flap (11) for simplifying manual handling of the second absorbent pad (3) during removal thereof from the first absorbent pad (2), wherein the gripping flap (11) is detached from the first absorbent pad (2), the method comprising the steps of
laminating a pre-cut second absorbent pad (3) on top of a first absorbent web (15);
releasably attaching a bottom surface of said pre-cut second absorbent pad (3) to a top surface of said first absorbent web (15); and
cutting out said first absorbent pad (2) from said first absorbent web (15), such as to form a stacked absorbent article (1) having first and second absorbent pads (2, 3) that are mutually releasably connected by means of fastening means, in particular pressure-sensitive adhesive, ultrasonic processing, hooks and loops, thermal bonding, mechanical bonding such as punching or embossing, or the like.

16. The method of claim 15, comprising the step of folding the at least one side portion before laminating the pre-cut second absorbent pad (3) on top of the first absorbent web (15).

17. Method for manufacturing an absorbent article (1), such as an incontinence pad, a sanitary towel, a panty liner or the like, having at least a first and a second stacked individual absorbent pads (2, 3), wherein the second absorbent pad (3) is provided with at least one side portion which has been folded upwardly and inwardly along a substantially longitudinal fold line (10), such as to form an easily graspable gripping flap (11) for simplifying manual handling of the second absorbent pad (3) during removal thereof from the first absorbent pad (2), wherein the gripping flap (11) is detached from the first absorbent pad (2), the method comprising the steps of
laminating a second absorbent web (14) on top of a first absorbent web (15);
releasably attaching a bottom surface of said second absorbent web (14) to a top surface of said first absorbent web (15); and
cutting out said first and second absorbent pads (2, 3) from said laminated absorbent webs, such as to form a stacked absorbent article (1) having first and second absorbent pads (2, 3) that are mutually releasably connected by means of fastening means, in particular pressure-sensitive adhesive, ultrasonic processing, hooks and loops, thermal bonding, mechanical bonding such as punching or embossing, or the like.

18. The method of claim 17, comprising the step of folding at least one lateral side of the second absorbent web (14) before laminating the folded second absorbent web (14) on top of the first absorbent web (15).

## Patentansprüche

1. Saugfähiger Artikel (1), wie zum Beispiel ein Inkontinenzpad, eine Damenbinde, ein Pantyliner oder Ähnliches, mit mindestens zwei gestapelten einzelnen saugfähigen Pads (2, 3), wobei ein erstes der saugfähigen Pads (2) für ein lösbares Anbringen an einer Unterwäsche eines Benutzers eingerichtet ist und eine untere Fläche eines zweiten der saugfähigen Pads (3) für ein lösbares Anbringen an einer oberen Fläche des ersten saugfähigen Pads (2) mittels eines Befestigungsmittels, insbesondere eines druckempfindlichen Haftmittels, Ultraschallbearbeitung, Haken und Schlaufen, thermischen Verbindens, mechanischen Verbindens, wie zum Beispiel Stanzen oder Prägen, oder Ähnliches eingerichtet ist, **dadurch gekennzeichnet, dass** das zweite saugfähige Pad (3) mindestens einen Seitenabschnitt aufweist, der entlang einer im Wesentlichen länglichen Faltlinie (10) nach oben und nach innen gefaltet worden ist, um so eine einfach greifbare Greiflasche (11) für eine vereinfachte manuelle Handhabung des zweiten saugfähigen Pads (3) während dessen Entfernens von dem ersten saugfähigen Pad (2) auszubilden, wobei die Greiflasche (11) von dem ersten saugfähigen Pad (2) getrennt ist.

2. Saugfähiger Artikel (1) nach Anspruch 1, **dadurch gekennzeichnet, dass** das erste saugfähige Pad (2) mindestens eine flüssigkeitsdurchlässige obere Schicht (5), eine flüssigkeitsundurchlässige Rückschicht (4) und einen dazwischen eingeschlossenen saugfähigen Kern (6) aufweist.

3. Saugfähiger Artikel (1) nach einem der vorstehenden Ansprüche, **dadurch gekennzeichnet, dass** das zweite saugfähige Pad (3) mindestens eine flüssigkeitsdurchlässige obere Schicht (8) aufweist.

4. Saugfähiger Artikel (1) nach Anspruch 3, **dadurch gekennzeichnet, dass** das zweite saugfähige Pad (3) zusätzlich eine flüssigkeitsdurchlässige Rückschicht (7) oder eine flüssigkeitsundurchlässige Rückschicht (7) oder eine semiflüssigkeitsdurchlässige Rückschicht (7) aufweist.

5. Saugfähiger Artikel (1) nach Anspruch 4, **dadurch gekennzeichnet, dass** das zweite saugfähige Pad (3) zusätzlich einen saugfähigen Kern aufweist, der zwischen der oberen Schicht (8) und Rückschicht (7) eingeschlossen ist.

6. Saugfähiger Artikel (1) nach einem der vorstehenden Ansprüche, **dadurch gekennzeichnet, dass** die Greiflasche (11) durch eine obere Schicht (8) des zweiten saugfähigen Pads (3) oder durch eine Rückschicht (7) des zweiten saugfähigen Pads (3) oder durch zumindest sowohl die obere Schicht (8) als auch die Rückschicht (7) des zweiten saugfähigen Pads (3) ausgebildet ist.

7. Saugfähiger Artikel (1) nach einem der vorstehenden Ansprüche, **dadurch gekennzeichnet, dass** die Greiflasche (11) mittels (9) eines Bands, einer Folie, eines Fadens und/oder einer Beschichtung verstärkt ist, was auf zumindest einen Teil einer inneren und/oder äußeren Fläche der Greiflasche (11) aufgebracht ist.

8. Saugfähiger Artikel (1) nach einem der vorstehenden Ansprüche, **dadurch gekennzeichnet, dass** die Greiflasche (11) flüssigkeitsundurchlässig ist und als Greiflasche (11) und seitliche Barriere zum Reduzieren eines seitlichen Austritts von Körperflüssigkeiten, wie zum Beispiel Menstruationsblut oder Urin, eine doppelte Funktionalität aufweist.

9. Saugfähiger Artikel (1) nach Anspruch 8, **dadurch gekennzeichnet, dass** die Flüssigkeitsundurchlässigkeit der Greiflasche (11) durch Aufbringen eines flüssigkeitsundurchlässigen Bands, Folie, Fadens und/oder Beschichtung auf zumindest einen Teil einer inneren und/oder äußeren Fläche der Greiflasche (11) oder durch Ausbilden der Greiflasche (11) durch zumindest eine flüssigkeitsundurchlässige Rückschicht (7) des zweiten saugfähigen Pads (3) umgesetzt ist.

10. Saugfähiger Artikel (1) nach einem der vorstehenden Ansprüche, **dadurch gekennzeichnet, dass** sich das erste saugfähige Pad (2) in einer Querrichtung in einem Bereich der mindestens einen Greiflasche (11) über eine Seitenkante des zweiten saugfähigen Pads (3) erstreckt, sodass auch das erste saugfähige Pad (2) während eines Entfernens des zweiten saugfähigen Pads (3) von dem ersten saugfähigen Pad (2) auf einfache Weise greifbar ist.

11. Saugfähiger Artikel (1) nach einem der vorstehenden Ansprüche, **dadurch gekennzeichnet, dass** sich das erste saugfähige Pad (2) entlang des gesamten Umfang des saugfähigen Artikels (1) über eine Seitenkante des zweiten saugfähigen Pads (3) erstreckt.

12. Saugfähiger Artikel (1) nach einem der vorstehenden Ansprüche, **dadurch gekennzeichnet, dass** der saugfähige Artikel (1) entlang einer ersten und einer zweiten seitlichen Faltlinie (13) gefaltet ist, die den saugfähigen Artikel (1) in drei vorzugsweise im Wesentlichen gleich große Teile zum Bereitstellen eines kleineren und tragbareren Artikels unterteilt, wobei das zweite saugfähige Pad (3) eine maximale Längserstreckung aufweist, die kleiner ist als die Hälfte der maximalen Längserstreckung des ersten saugfähigen Pads (2) und eingerichtet ist, in einen zentralen Bereich des saugfähigen Artikels (1) zu passen, ohne entlang der seitlichen Faltlinien (13) gefaltet zu sein.

13. Saugfähige Artikel (1) nach einem der vorstehenden Ansprüche 1-9, **dadurch gekennzeichnet, dass** eine Seitenkante des ersten saugfähigen Pads (2) die Seitenkante des zweiten saugfähigen Pads (3) entlang des gesamten Umfangs der saugfähigen Pads (2, 3) überlappt.

14. Saugfähiger Artikel (1) nach einem der vorstehenden Ansprüche, **dadurch gekennzeichnet, dass** das erste saugfähige Pad (2) zwei beabstandete Flügelabschnitte (12) aufweist, die sich über die seitlichen gegenüberliegenden Seiten des saugfähigen Kerns (6) des ersten saugfähigen Pads (2) erstrecken, wobei jeder der Flügelabschnitte (12) eingerichtet ist, um um eine Seitenkante des Schrittabschnitts der Unterwäsche zum Sichern des ersten saugfähigen Pads (2) an der Unterwäsche und Vorbeugen gegen dessen Verschmutzung gefaltet zu werden.

15. Verfahren zum Herstellen eines saugfähigen Artikels (1), wie zum Beispiel ein Inkontinenzpad, eine Damenbinde, ein Pantyliner oder Ähnliches, mit mindestens einem ersten und einem zweiten gestapelten einzelnen saugfähigen Pad (2, 3), wobei das zweite saugfähige Pad (3) mit mindestens einem Seitenabschnitt bereitgestellt ist, der entlang einer im Wesentlichen länglichen Faltlinie (10) nach oben und nach innen gefaltet worden ist, um zum so eine einfach greifbare Greiflasche (11) für eine vereinfachte manuelle Handhabung des zweiten saugfähigen Pads (3) während dessen Entfernens von dem ersten saugfähigen Pad (2) auszubilden, wobei die Greiflasche (11) von dem ersten saugfähigen Pad (2) getrennt ist und das Verfahren die Schritte umfasst:
Laminieren eines vorgeschnittenen zweiten saugfähigen Pads (3) auf eine erste saugfähige Bahn (15);
lösbares Anbringen einer unteren Fläche des vorgeschnittenen zweiten saugfähigen Pads (3) an einer oberen Fläche der ersten saugfähigen Bahn (15); und
Ausschneiden des ersten saugfähigen Pads (2) aus der ersten saugfähigen Bahn (15), um so einen gestapelten saugfähigen Artikel (1) mit einem ersten und zweiten saugfähigen Pad (2, 3) auszubilden, die mittels eines Befestigungsmittels, insbesondere eines druckempfindlichen Haftmittels, einer Ultraschallbearbeitung, Haken und Schleifen, thermischen Verbindens, mechanischen Verbindens, wie zum Beispiel Stanzen oder Prägen, oder Ähnliches miteinander lösbar verbunden werden.

16. Verfahren nach Anspruch 15, mit dem Schritt eines Faltens des mindestens einen Seitenabschnitt vor einem Laminieren des vorgeschnittenen zweiten saugfähigen Pads (3) auf die erste saugfähige Bahn (15).

17. Verfahren zum Herstellen eines saugfähigen Artikels (1), wie zum Beispiel ein Inkontinenzpad, eine Damenbinde, ein Pantyliner oder Ähnliches, mit mindestens einem ersten und zweiten gestapelten einzelnen saugfähigen Pad (2, 3), wobei das zweite saugfähige Pad (3) mit mindestens einem Seitenabschnitt bereitgestellt ist, der entlang einer im Wesentlichen länglichen Faltlinie (10) nach oben und nach innen gefaltet worden ist, um so eine einfach greifbare Greiflasche (11) für eine vereinfachte manuelle Handhabung des zweiten saugfähigen Pads (3) während dessen Entfernens von dem ersten saugfähigen Pad (2) auszubilden, wobei die Greiflasche (11) von dem ersten saugfähigen Pad (2) getrennt ist, und das Verfahren die Schritte umfasst:
Laminieren einer zweiten saugfähigen Bahn (14) auf eine erste saugfähige Bahn (15);
lösbares Anbringen einer unteren Fläche der zweiten saugfähigen Bahn (14) auf einer oberen Fläche der ersten saugfähigen Bahn (15); und
Ausschneiden des ersten und zweiten saugfähigen Pads (2, 3) aus den laminierten saugfähigen Bahnen, um so einen gestapelten saugfähigen Artikel (1) mit einem ersten und zweiten saugfähigen Pad (2, 3) auszubilden, die mittels eines Befestigungsmittels, insbesondere eines druckempfindlichen Haftmittels, einer Ultraschallbearbeitung, Haken und Schlaufen, thermischen Verbindens, mechanischen Verbindens, wie zum Beispiel Stanzen oder Prägen, oder Ähnliches miteinander lösbar verbunden werden.

18. Verfahren nach Anspruch 17, mit dem Schritt eines Faltens von mindestens einer seitlichen Seite der zweiten saugfähigen Bahn (14) vor einem Laminieren der gefalteten zweiten saugfähigen Bahn (14) auf der ersten saugfähigen Bahn (15).

## Revendications

1. Article absorbant (1), tel qu'une serviette d'incontinence, une serviette hygiénique, un protège-slip ou un article similaire, comprenant au moins deux coussinets absorbants individuels superposés (2, 3), un premier desdits coussinets absorbants (2) étant configuré pour être fixé de façon amovible à un sous-vêtement d'un utilisateur, et une surface inférieure d'un deuxième desdits coussinets absorbants (3) étant configurée pour être fixée de façon amovible à une surface supérieure dudit premier coussinet absorbant (2) par un moyen de fixation, en particulier un adhésif sensible à la pression, un traitement ultrasonique, des crochets et des boucles, un thermocollage, un liage mécanique tel que le poinçonnage ou le gaufrage, ou un procédé similaire, **caractérisé en ce que** le deuxième coussinet absorbant (3) comprend au moins une partie latérale qui a été repliée vers le haut et vers l'intérieur le long d'une ligne de pliage sensiblement longitudinale (10), de façon à former un rabat de préhension facile à saisir (11) pour simplifier la manipulation manuelle du deuxième coussinet absorbant (3) durant le retrait de celui-ci du premier coussinet absorbant (2), le rabat de préhension (11) étant détaché du premier coussinet absorbant (2).

2. Article absorbant (1) selon la revendication 1, **caractérisé en ce que** le premier coussinet absorbant (2) comprend au moins une feuille supérieure perméable aux liquides (5), une feuille postérieure imperméable aux liquides (4) et une partie centrale absorbante (6) incluse entre celles-ci.

3. Article absorbant (1) selon l'une quelconque des revendications précédentes, **caractérisé en ce que** le deuxième coussinet absorbant (3) comprend au moins une feuille supérieure perméable aux liquides (8).

4. Article absorbant (1) selon la revendication 3, **caractérisé en ce que** le deuxième coussinet absorbant (3) comprend en outre une feuille postérieure perméable aux liquides (7), ou une feuille postérieure imperméable aux liquides (7), ou une feuille postérieure semi-perméable aux liquides (7).

5. Article absorbant (1) selon la revendication 4, **caractérisé en ce que** le deuxième coussinet absorbant (3) comprend en outre une partie centrale absorbante incluse entre la feuille supérieure (8) et la feuille postérieure (7).

6. Article absorbant (1) selon l'une quelconque des revendications précédentes, **caractérisé en ce que** le rabat de préhension (11) est formé par une feuille supérieure (8) du deuxième coussinet absorbant (3), ou par une feuille postérieure (7) du deuxième coussinet absorbant (3), ou par au moins la feuille supérieure (8) ainsi que la feuille postérieure (7) du deuxième coussinet absorbant (3).

7. Article absorbant (1) selon l'une quelconque des revendications précédentes, **caractérisé en ce que** le rabat de préhension (11) est renforcé au moyen (9) d'un ruban, d'un film, d'un fil et/ou d'un revêtement appliqué sur au moins une partie d'une surface interne et/ou externe du rabat de préhension (11).

8. Article absorbant (1) selon l'une quelconque des revendications précédentes, **caractérisé en ce que** le rabat de préhension (11) est imperméable aux liquides et remplit une double fonction de rabat de préhension (11) et de barrière latérale servant à réduire les fuites latérales de liquides corporels, tels que le sang menstruel ou l'urine.

9. Article absorbant (1) selon la revendication 8, **caractérisé en ce que** l'imperméabilité aux liquides du rabat de préhension (11) est réalisée par application d'un ruban, d'un film, d'un fil et/ou d'un revêtement imperméable aux liquides sur au moins une partie d'une surface interne et/ou externe du rabat de préhension (11), ou par formation du rabat de préhension (11) à partir d'au moins une feuille postérieure imperméable aux liquides (7) du deuxième coussinet absorbant (3).

10. Article absorbant (1) selon l'une quelconque des revendications précédentes, **caractérisé en ce que** le premier coussinet absorbant (2) s'étend au-delà d'un bord latéral dudit deuxième coussinet absorbant (3) dans une direction transversale dans une région dudit au moins un rabat de préhension (11), de telle sorte que le premier coussinet absorbant (2) soit lui aussi facile à saisir durant le retrait du deuxième coussinet absorbant (3) du premier coussinet absorbant (2).

11. Article absorbant (1) selon l'une quelconque des revendications précédentes, **caractérisé en ce que** le premier coussinet absorbant (2) s'étend au-delà d'un bord latéral dudit deuxième coussinet absorbant (3) sur toute la périphérie dudit article absorbant (1).

12. Article absorbant (1) selon l'une quelconque des revendications précédentes, **caractérisé en ce que** l'article absorbant (1) est plié le long d'une première ligne de pliage latérale et d'une deuxième ligne de pliage latérale (13) qui divisent l'article absorbant (1) en trois parties, préférablement de taille sensiblement égale, pour produire un article plus petit et plus facile à transporter, le deuxième coussinet absorbant (3) ayant une extension longitudinale maximale qui est inférieure à la moitié de l'extension longitudinale maximale du premier coussinet absorbant (2) et étant agencé pour tenir dans une région centrale dudit article absorbant (1) sans être plié le long desdites lignes de pliage latérales (13).

13. Article absorbant (1) selon l'une quelconque des revendications précédentes 1 à 9, **caractérisé en ce qu'**un bord latéral du premier coussinet absorbant (2) chevauche le bord latéral du deuxième coussinet absorbant (3) sur toute la périphérie des coussinets absorbants (2, 3).

14. Article absorbant (1) selon l'une quelconque des revendications précédentes, **caractérisé en ce que** le premier coussinet absorbant (2) comporte deux sections espacées formant des ailes (12) qui s'étendent au-delà des côtés latéralement opposés de la partie centrale absorbante (6) du premier coussinet absorbant (2), chacune desdites sections formant des ailes (12) étant agencée pour être repliée autour d'un bord latéral de la partie du sous-vêtement correspondant à l'entrejambe, pour fixer le premier coussinet absorbant (2) au sous-vêtement, en empêchant ainsi celui-ci d'être sali.

15. Procédé de fabrication d'un article absorbant (1), tel qu'une serviette d'incontinence, une serviette hygiénique, un protège-slip ou un article similaire, comportant au moins un premier coussinet absorbant et un deuxième coussinet absorbant individuels superposés (2, 3), le deuxième coussinet absorbant (3) étant pourvu d'au moins une partie latérale qui a été repliée vers le haut et vers l'intérieur le long d'une ligne de pliage sensiblement longitudinale (10), de façon à former un rabat de préhension facile à saisir (11) pour simplifier la manipulation manuelle du deuxième coussinet absorbant (3) durant le retrait de celui-ci du premier coussinet absorbant (2), le rabat de préhension (11) étant détaché du premier coussinet absorbant (2), le procédé comprenant les étapes qui consistent à :
appliquer par stratification un deuxième coussinet absorbant prédécoupé (3) sur le dessus d'une première nappe absorbante (15) ;
fixer de façon amovible une surface inférieure dudit deuxième coussinet absorbant prédécoupé (3) à une surface supérieure de ladite première nappe absorbante (15) ; et
découper ledit premier coussinet absorbant (2) dans ladite première nappe absorbante (15) et l'enlever de celle-ci, de façon à former un article absorbant stratifié (1) comportant un premier coussinet absorbant et un deuxième coussinet absorbant (2, 3) qui sont connectés l'un à l'autre de façon amovible par un moyen de fixation, en particulier un adhésif sensible à la pression, un traitement ultrasonique, des crochets et des boucles, un thermocollage, un liage mécanique tel que le poinçonnage ou le gaufrage, ou un procédé similaire.

16. Procédé selon la revendication 15, comprenant l'étape de pliage de ladite au moins une partie latérale avant l'application par stratification du deuxième coussinet absorbant prédécoupé (3) sur le dessus de la première nappe absorbante (15).

17. Procédé de fabrication d'un article absorbant (1), tel qu'une serviette d'incontinence, une serviette hygiénique, un protège-slip ou un article similaire, comportant au moins un premier coussinet absorbant et un deuxième coussinet absorbant individuels superposés (2, 3), le deuxième coussinet absorbant (3) étant pourvu d'au moins une partie latérale qui a été repliée vers le haut et vers l'intérieur le long d'une ligne de pliage sensiblement longitudinale (10), de façon à former un rabat de préhension facile à saisir (11) pour simplifier la manipulation manuelle du deuxième coussinet absorbant (3) durant le retrait de celui-ci du premier coussinet absorbant (2), le rabat de préhension (11) étant détaché du premier coussinet absorbant (2), le procédé comprenant les étapes qui consistent à :
appliquer par stratification une deuxième nappe absorbante (14) sur le dessus d'une première nappe absorbante (15) ;
fixer de façon amovible une surface inférieure de ladite deuxième nappe absorbante (14) à une surface supérieure de ladite première nappe absorbante (15) ; et
découper lesdits premier coussinet absorbant et deuxième coussinet absorbant (2, 3) dans lesdites nappes absorbantes stratifiées, de façon à former un article absorbant stratifié (1) comportant un premier coussinet absorbant et un deuxième coussinet absorbant (2, 3) qui sont connectés l'un à l'autre de façon amovible par un moyen de fixation, en particulier un adhésif sensible à la pression, un traitement ultrasonique, des crochets et des boucles, un thermocollage, un liage mécanique tel que le poinçonnage ou le gaufrage, ou un procédé similaire.

18. Procédé selon la revendication 17, comprenant l'étape qui consiste à replier au moins un côté latéral de la deuxième nappe absorbante (14) avant l'application par stratification de la deuxième nappe absorbante repliée (14) sur le dessus de la première nappe absorbante (15).
